# EUROPEAN PATENT APPLICATION

(11) **EP 4 223 353 A1**
(43) Date of publication of application: **09.08.2023**
(21) Application number: 21875623.7
(22) Date of filing: 28.09.2021
(51) Int. Cl.: A61M 37/00

(54) **PART WITH NEEDLE-LIKE PROJECTIONS**

(30) Priority: 30.09.2020 JP 2020166213
(71) Applicant: Shiseido Company, Ltd., Chuo-ku Tokyo 104-0061 (JP); Asti Corporation, Hamamatsu-shi, Shizuoka 432-8056 (JP)
(72) Inventor: OBANA, Takakazu, Tokyo 104-0061 (JP); SHIMIZU, Hiroko, Tokyo 104-0061 (JP); MIYAZAWA, Kazuyuki, Tokyo 104-0061 (JP); OGAI, Noriyuki, Hamamatsu-shi, Shizuoka 432-8056 (JP); TODA, Yasuhiro, Hamamatsu-shi, Shizuoka 432-8056 (JP); INOH, Akinori, Nisshin-shi, Aichi 470-0135 (JP)
(74) Representative: Ter Meer Steinmeister & Partner
(86) International application number: PCT/JP2021/035658
(87) International publication number: WO 2022/071322

(57) **Abstract**

A needle-shaped projection-including part (1), which is configured to inject a liquid to a target object, the needle-shaped projection-including part including: a plate (111) in which a vertically penetrating discharge hole (114) is formed; one or a plurality of fine needle-shaped projections (112) that project from one face of the plate (111); and a lip portion (113) that is formed at an outer edge portion of the one face of the plate (111), wherein in response to pushing out of the liquid from the discharge hole (114), the liquid spreads in a space defined by the target object, the lip portion (113), and the one face of the plate (111), and the liquid enters the target object through holes formed by the one or plurality of needle-shaped projections (112).

## Description

### [Technical Field]

The present invention relates to a needle-shaped projection-including part, in particular, to a needle-shaped projection-including part that is to be attached to the front end of a syringe barrel or mounted in a needle-fixable injector.

### [Background Art]

Traditionally, multi-needle plates for a syringe having a plurality of needles (multi-needles, syringe needle mechanisms) are known.

In recent years, also known are microneedle masks including dissolvable microneedles that are to be attached to skin and left to stand, thereby penetrating beauty ingredients into the skin (see, for example, PTL 1).

Meanwhile, PTL 2 proposes an injector including a shield that becomes conically wider towards the outside of needles in order to stretch skin before puncture.

[Citation List] JP Laid-Open Patent Publication No. 2017-051354 [PTL 2] JP Patent No. 4746818 [Summary of Invention]/[Technical Problem]

Because the microneedles used with the mask as described in PTL 1 are integrally formed with the beauty ingredients, it is necessary for the microneedles to be left to stand for a long time in order to inject the beauty ingredients dissolved in the microneedles, into the skin. Also, such beauty ingredients that can be integrally formed with the microneedles are limited.

Moreover, provision of a shield as a separate body, as described in PTL 2, raises a risk that a drug solution overflown from the holes in the skin enters the gap between the shield and the device body. This is likely to reduce the quantity of the drug solution to be applied.

In view of the foregoing, it is an object of the present invention to provide a needle-shaped projection-including part that is able to retain a liquid composition/cosmetic liquid containing any active ingredient on and in a target object so as not to leak from the gap.

### [Solution to Problem]

In order to solve the above problem, one aspect of the present invention is a needle-shaped projection-including part, which is configured to inject a liquid to a target object, including:
a plate in which a vertically penetrating discharge hole is formed;
one or a plurality of fine needle-shaped projections that project from one face of the plate; and
a lip portion that is formed at an outer edge portion of the one face of the plate,
wherein in response to the liquid being discharged through the discharge hole, the liquid spreads in a space defined by the target object, the lip portion, and the one face of the plate, and the liquid enters the target object through holes formed by the one or plurality of needle-shaped projections.

### [Advantageous Effects of Invention]

According to one aspect, the needle-shaped projection-including part is able to retain a liquid composition/cosmetic liquid containing any active ingredient on and in a target object so as not to leak from the gap.

### [Brief Description of Drawings]

FIG. 1 is an overall view of a syringe set in which a needle-shaped projection-including part according to a first embodiment of the present invention is attached to a syringe barrel.
FIG. 2 is a cross-sectional view of the syringe set of FIG. 1.
FIG. 3 is an exploded view of the syringe set of FIG. 1, in which a syringe barrel is separated, and a plate portion and an attaching frame in the needle-shaped projection-including part are also separated.
FIG. 4 is a cross-sectional perspective view of a needle-shaped projection-including part according to the first embodiment.
FIG. 5A is a view of a state where a target object is punctured by a needle-shaped projection-including part according to the first embodiment.
FIG. 5B is a view of a state where a liquid is injected into a target object from a needle-shaped projection-including part according to the first embodiment.
FIG. 6 is a cross-sectional view of a needle-shaped projection-including part according to a second embodiment.
FIG. 7A is a perspective view of the bottom face of the needle-shaped projection-including part of
FIG. 6.

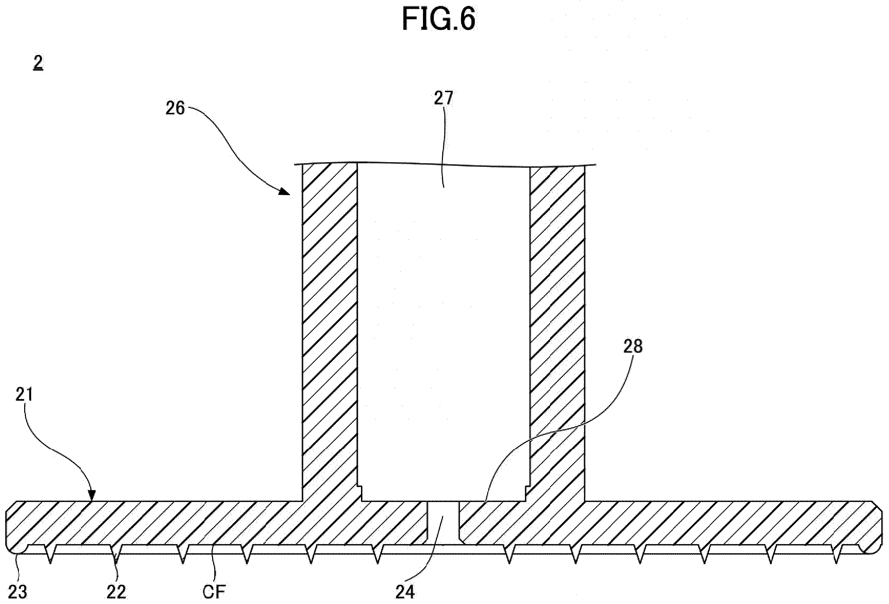

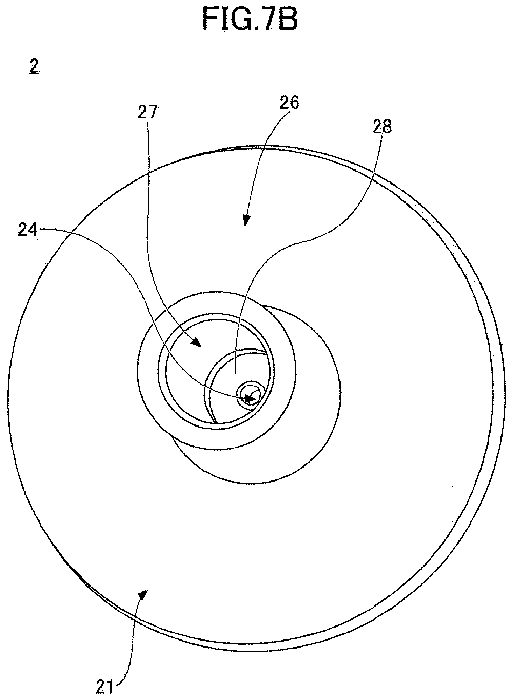

FIG.7B is perspective view of the top face of the needle-shaped projection-including part of FIG.6.

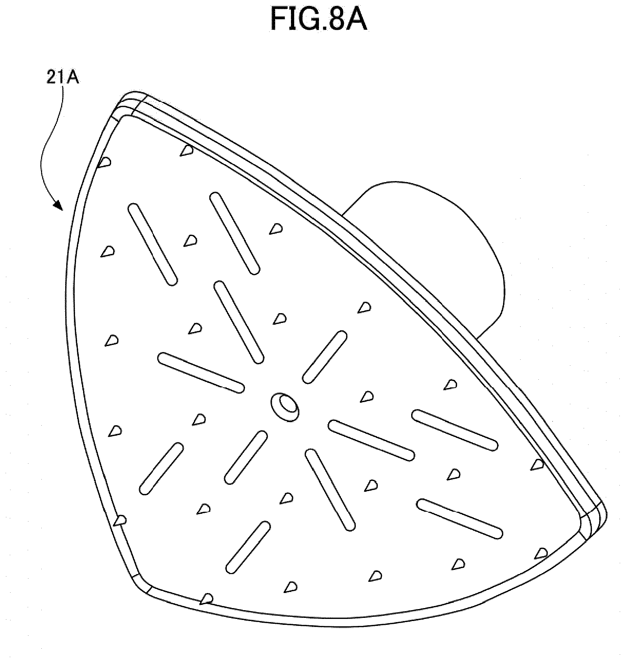

FIG. 8A is a perspective view of the bottom face of a modified example of a needle plate of a needle-shaped projection-including part according to the second embodiment.

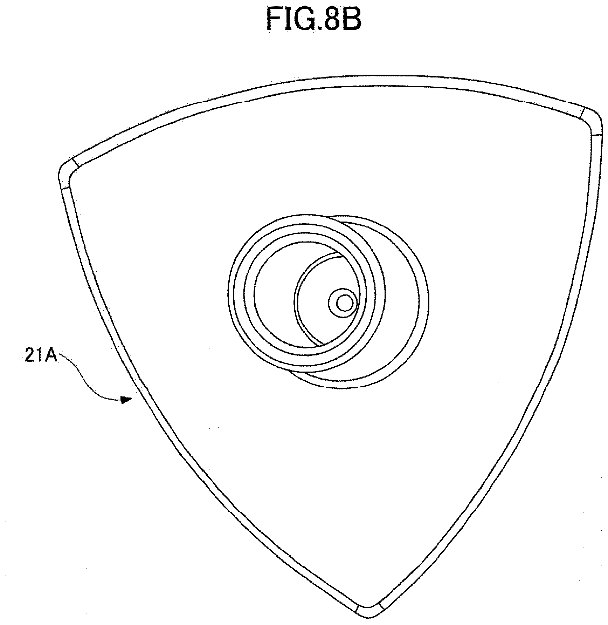

FIG.8B perspective view of the top face of the needle-shaped projection-including part of FIG.8A.

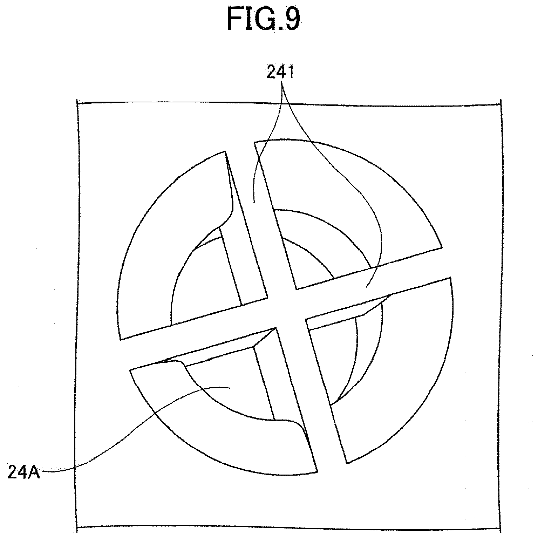

FIG. 9 is a view of a modified example of a central hole of a needle-shaped projection-including part of the present invention.

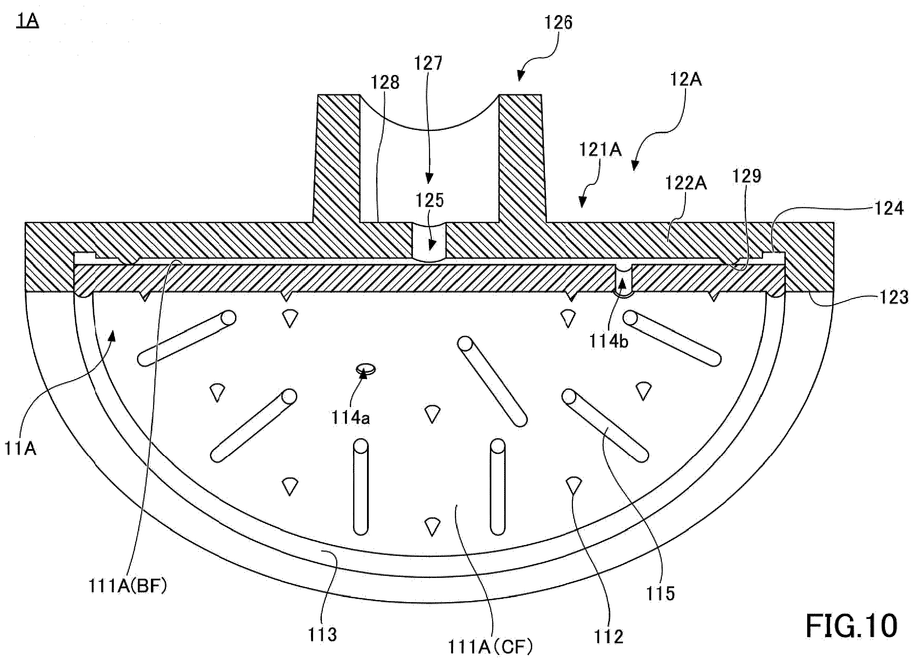

FIG. 10 is a cross-sectional perspective view of a needle-shaped projection-including part according to a third embodiment of the present invention.

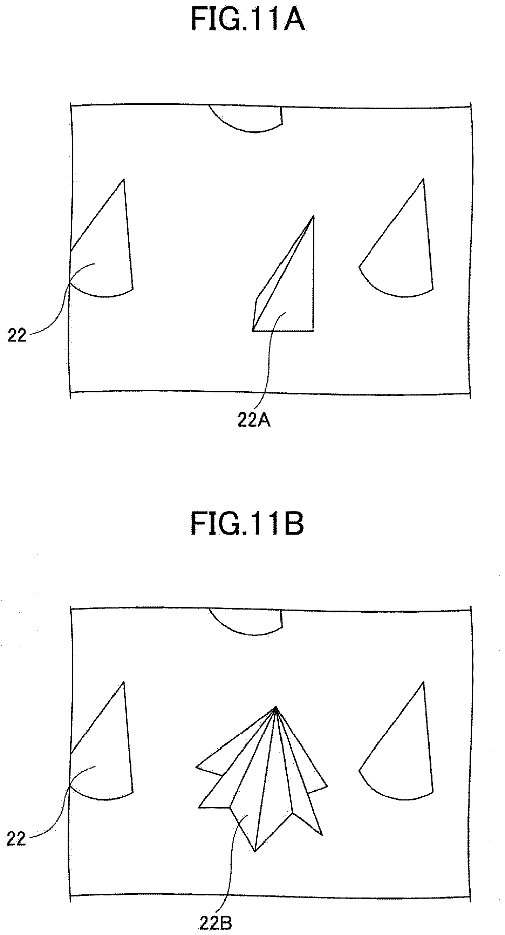

FIG. 11A a view of a needle-shaped projection having a polygonal pyramid shape, as modified example of the needle-shaped projection of a needle-shaped projection-including part of the present invention.
FIG. 11B a view of a needle-shaped projection having a conical pentagram shape, as modified example of the needle-shaped projection of a needle-shaped projection-including part of the present invention.

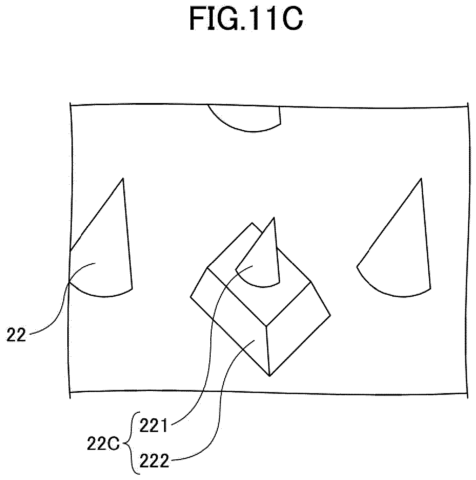

FIG. 11C is a view of a two-stepped needle-shaped projection of a base and a top needle, as a modified example of the needle-shaped projection of a needle-shaped projection-including part of the present invention.

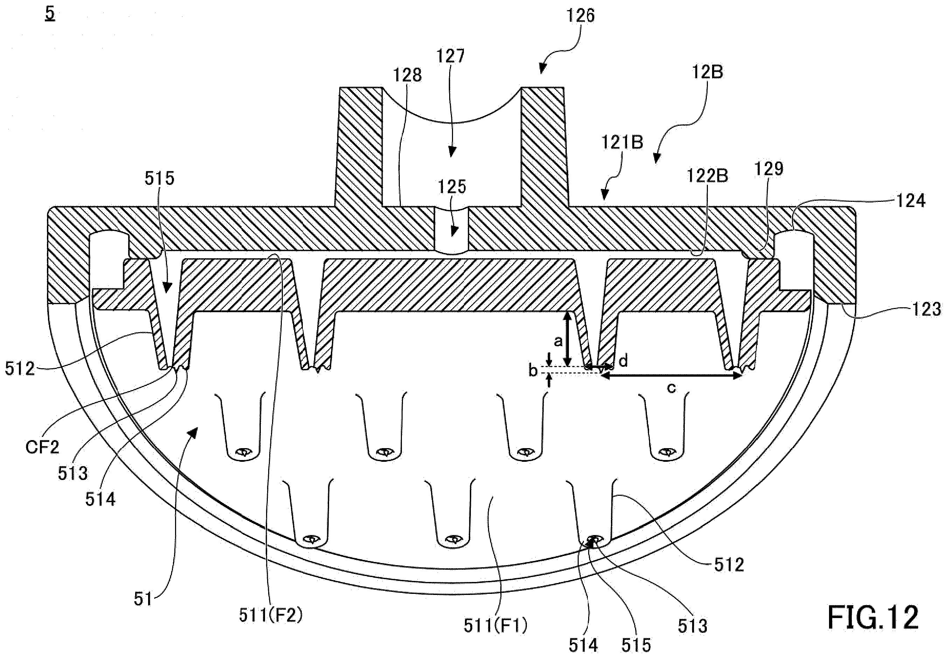

FIG. 12 is a cross-sectional perspective view of a needle-shaped projection-including part according to a fourth embodiment of the present invention.

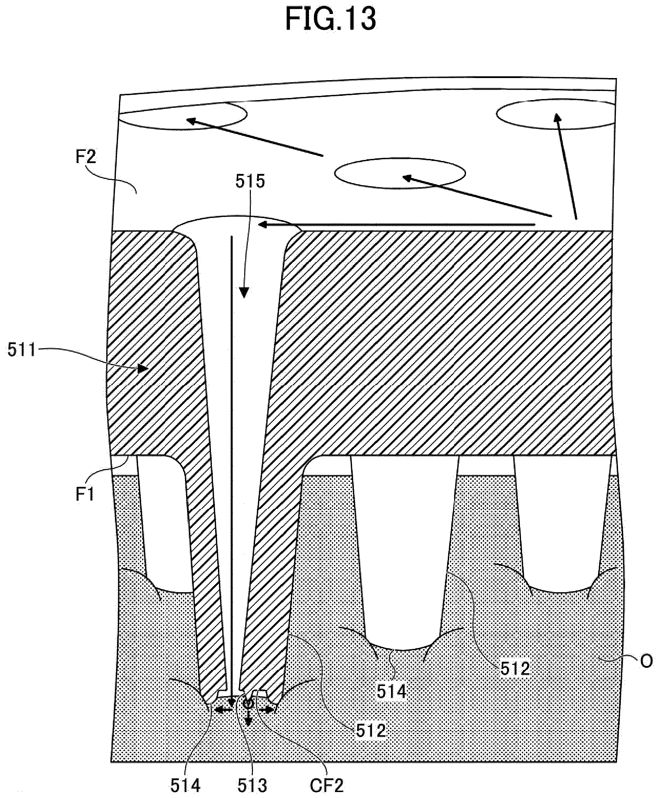

FIG. 13 is a view of a state where a liquid is injected into a target object from a needle plate of a needle-shaped projection-including part according to the fourth embodiment.

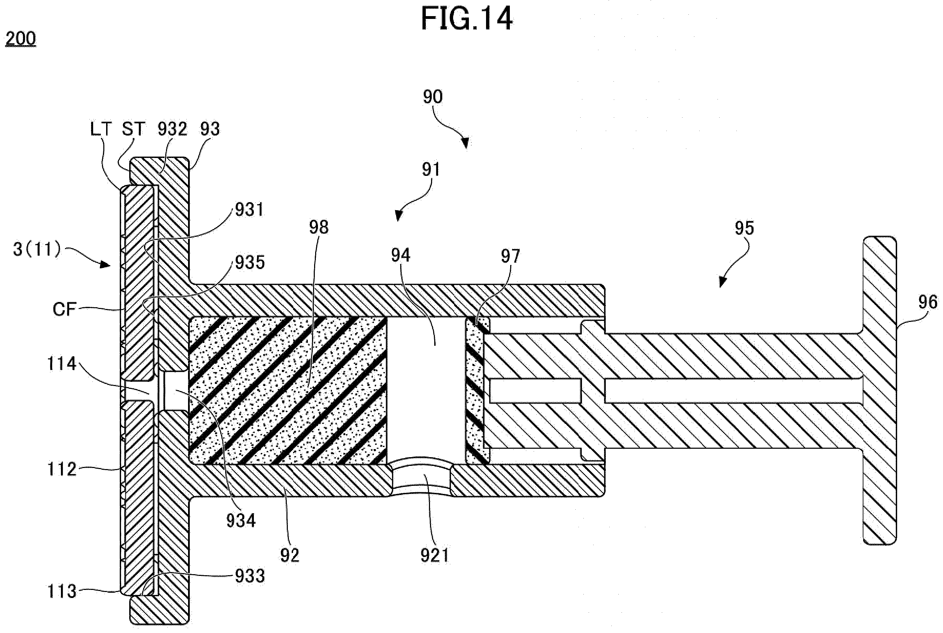

FIG. 14 is an overall cross-sectional view of a needle-fixable injector in which a needle-shaped projection-including part according to a fifth embodiment of the present invention is mounted.

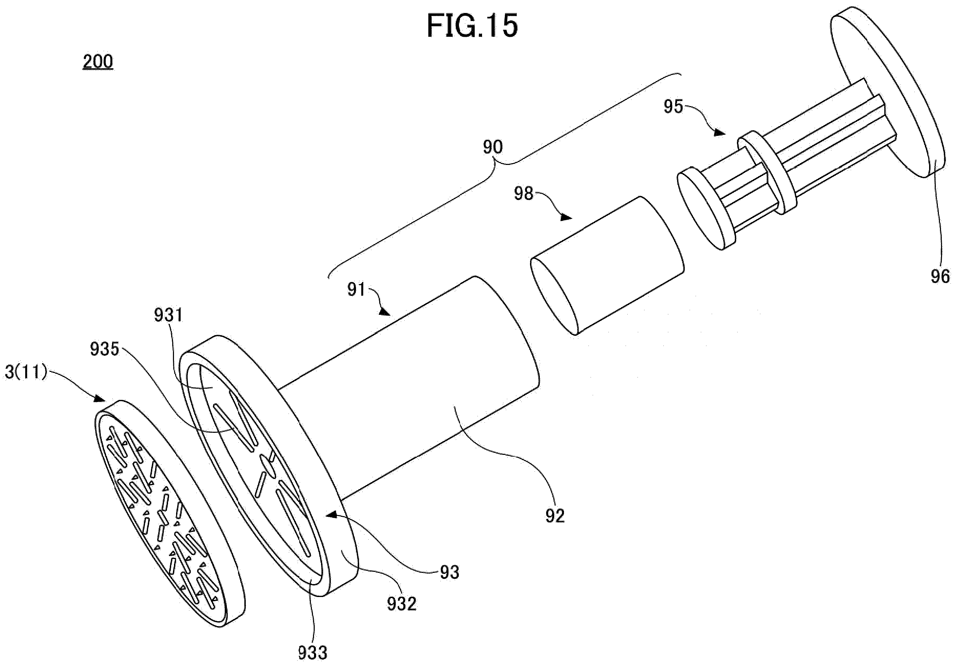

FIG. 15 is an exploded perspective view of the needle-fixable injector of FIG. 14.

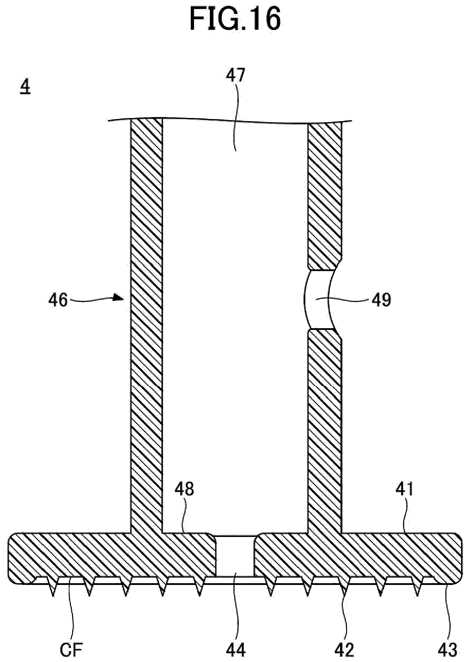

FIG. 16 is a view of a needle-fixable injector in which a head portion of an outer barrel and a needle plate are integrally formed.

### [Description of Embodiments]

Hereinafter, embodiments of the present invention will be described with reference to the drawings. In the following, the same components across the drawings are given the same symbols, and duplicate description may be omitted.

The present invention relates to a needle-shaped projection-including part, in particular, to a part including one or a plurality of fine needle-shaped projections, where the part is to be attached to the front end of a syringe barrel, mounted in a needle-fixable injector, attached to a cosmetic container such as a dial pen, a knock pen, a tube, or a pouch, and mounted in a needle ejection-type inj ector.

In the needle-shaped projection-including part of the present invention, for example, a beauty ingredient as the liquid is applied to skin as the target object. Also, the needle-shaped projection-including part is mainly for beauty applications and therapeutic applications, and is applied to puncture the skin.

### «Syringe to which a needle-shaped projection plate is attached»

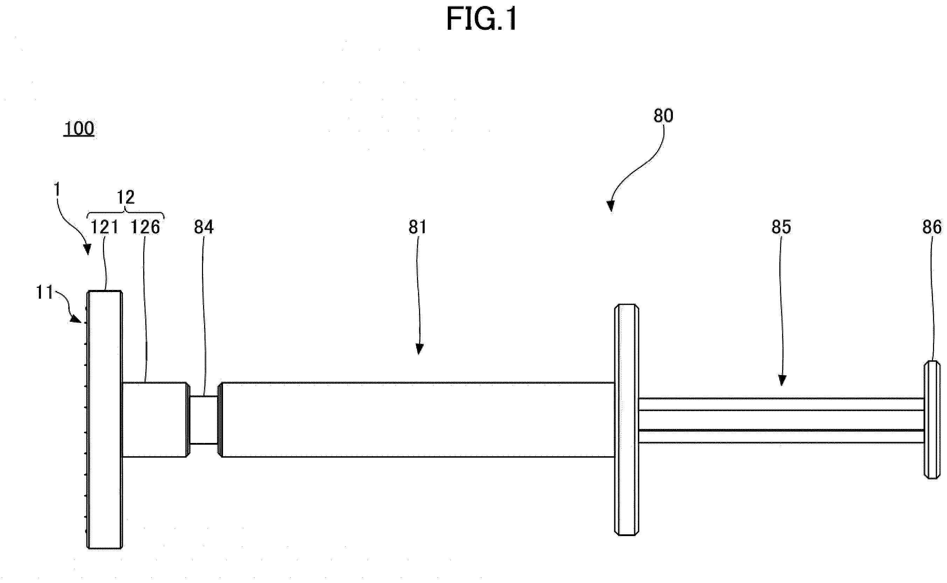

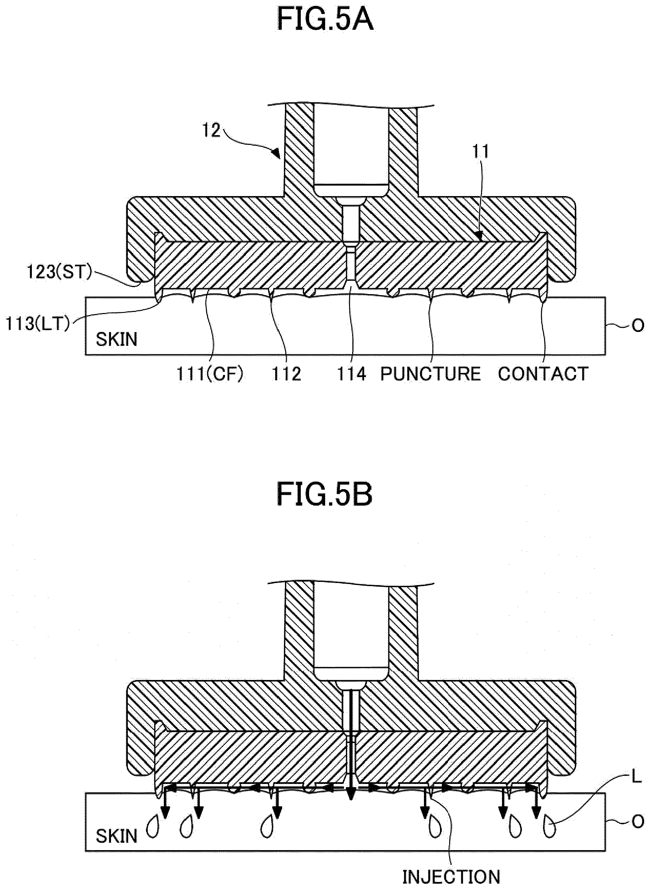

Referring to FIGS. 1 to 5B, a syringe set, in which a needle-shaped projection-including part according to a first embodiment of the present invention and a syringe barrel are coupled to each other, will be described. First, referring to FIGS. 1 to 3, the overall configuration of the syringe set according to the first embodiment will be described.

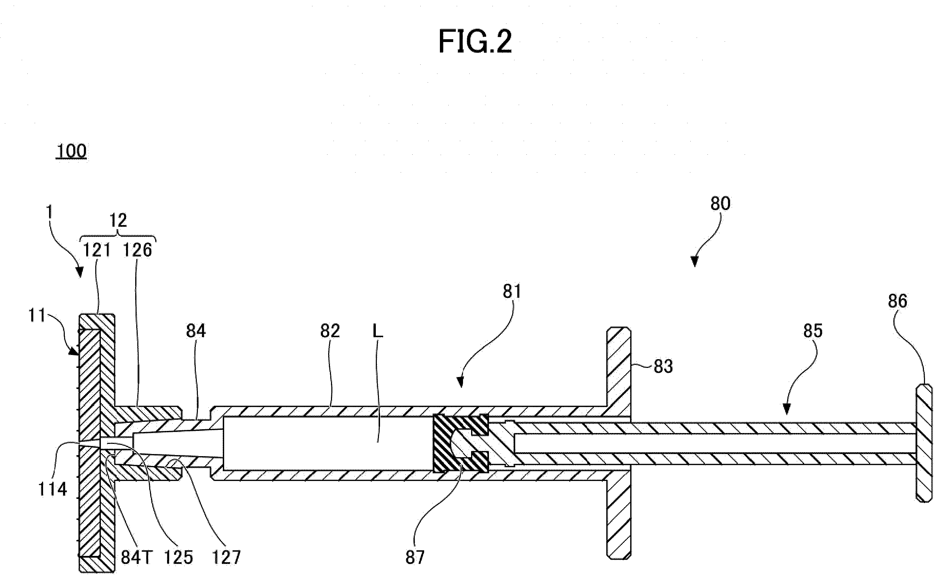

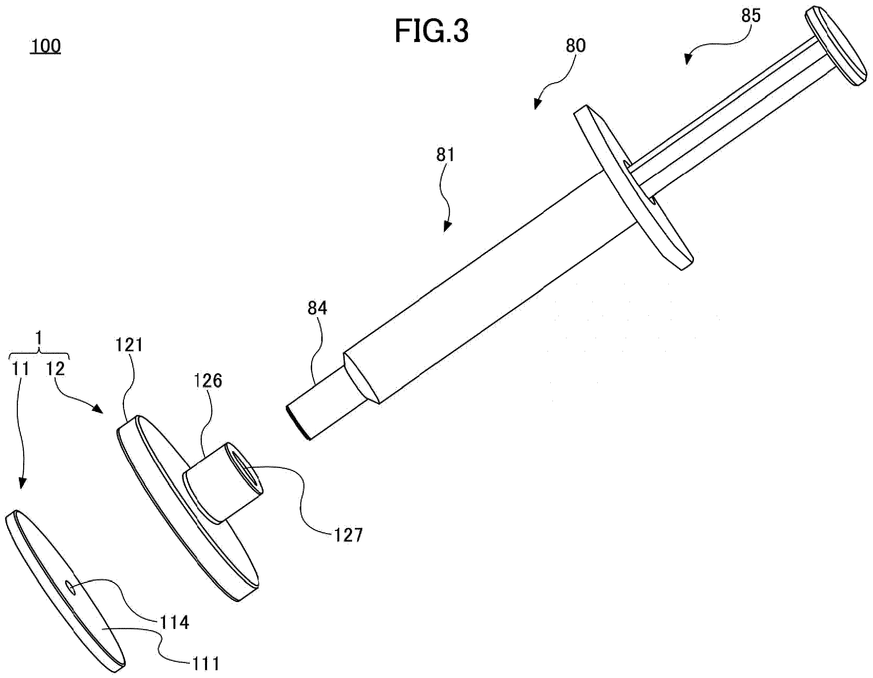

FIG. 1 is an overall view of a syringe set 100 in which a needle-shaped projection-including part according to the first embodiment of the present invention is attached to a syringe barrel. FIG. 2 is a cross-sectional view of the syringe set 100 of FIG. 1. FIG. 3 is an exploded view of the syringe set 100 of FIG. 1, in which a syringe 80 is separated, and a needle plate 11 and an attaching frame 12 in a needle-shaped projection-including part 1 are also separated.

As illustrated in FIG. 2, the syringe 80 includes a syringe barrel (syringe body, cylinder) 81 and a pusher (plunger) 85. The syringe barrel 81 houses liquid content L, and the pusher 85 is inserted into the syringe barrel 81. Specifically, the syringe barrel 81 includes an outer barrel portion 82 housing the content L, a flange 83 provided at the back end of the outer barrel portion 82, and a front barrel portion 84 as the front-end portion.

Here, the syringe 80, to which the needle-shaped projection-including part 1 of the present invention is to be attached, may be a syringe having a volume of, for example, 1 mL, 2.5 mL, 5 mL, or 10 mL. In accordance with a specific manner in which the needle-shaped projection-including part 1 is used, the syringe may have a larger or smaller volume.

In the present embodiment, instead of one hollow syringe needle that is typically used, the needle-shaped projection-including part (needle assembly, needle-shaped projection-including component) 1 is used and coupled to the front barrel portion 84 of the syringe barrel 81.

Specifically, the needle-shaped projection-including part 1 includes a cylindrical attaching barrel 126 that is fitted over the front barrel portion 84 of the syringe barrel 81. When the needle-shaped projection-including part 1 is attached to the syringe 80, the front barrel portion 84 of the syringe barrel 81 is inserted into and engaged with a fitting hole 127 of the attaching barrel 126 of the needle-shaped proj ection-including part. It may be possible to use a syringe having a structure that locks so that the attaching barrel 126 is not readily detached from the front barrel portion 84 of the syringe barrel 81. Also, the syringe may have a shape stipulated according to such standards as ISO (International Organization for Standardization) and JIS (Japanese Industrial Standards).

Here, the needle-shaped projection applied in the needle-shaped projection-including part 1 of the present invention is a solid needle that forms the needle-shaped fine projection. The needle-shaped projection preferably has a diameter in the range of from 2 µm through 5000 µm, and more preferably in the range of from 5 µm through 3000 µm, as the diameter of the thickest portion of the needle.

In the syringe 80, as illustrated in FIG. 2, the pusher 85 is pushed in response to pushing of a pushing part 86 at the back end, and moved in the outer barrel portion 82 of the syringe barrel 81, whereby the content L is discharged from the syringe barrel 81 into the needle-shaped projection-including part 1.

Once the content L has reached the needle-shaped projection-including part 1, the content is sent to the target object, such as skin, through a flow path 125 and a discharge hole 114.

Referring to FIG. 2, a gasket 87 may adhere to the front-end portion of the pusher 85, where the gasket is a thin-plate packing (hermetically sealing element) made of rubber or elastomer for preventing liquid leakage and contamination. Note that, the packing may or may not be provided.

Moreover, in order to protect a plurality of needle-shaped projections 112 of the needle-shaped projection-including part 1 of the present invention, a cap configured to cover the needle-shaped projection-including part 1 may be attached.

### <First embodiment of the needle-shaped projection-including part>

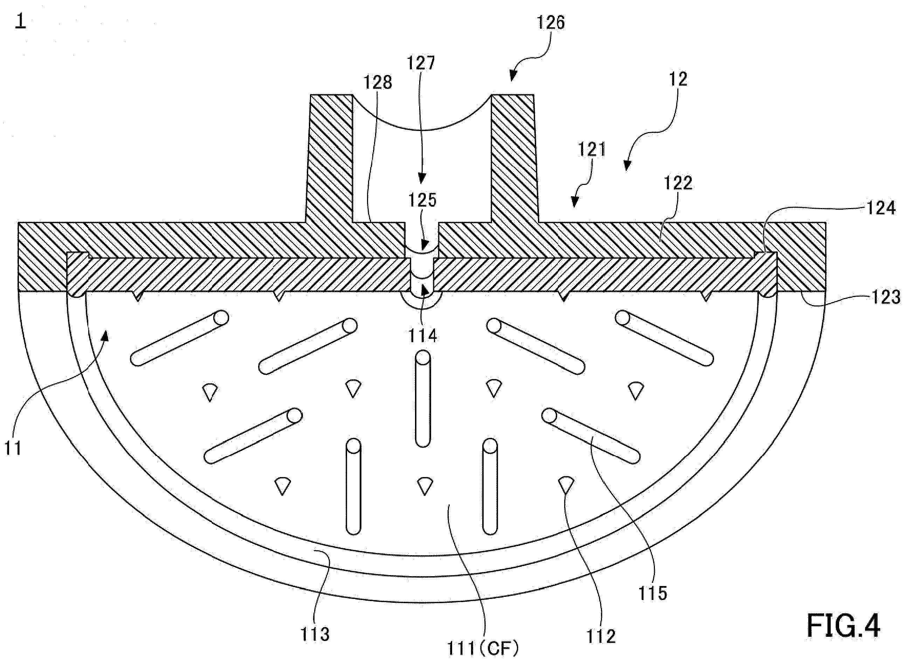

FIG. 4 is an enlarged cross-sectional perspective view of the needle-shaped projection-including part 1 according to the first embodiment.

In the present embodiment, the needle-shaped projection-including part 1 is an assembled body (assembly) of two members: a needle plate 11 and an attaching frame 12.

In the needle plate 11, a plate body 111 having a circular flat-plate shape is provided with a plurality of needle-shaped projections 112 and a lip portion 113. The plate body 111 has a discharge hole 114 at the center thereof. The plate body 111 serves as a plate.

In the needle plate 11, the plurality of needle-shaped projections 112 project from a front-end face CF of the plate body 111. The plurality of needle-shaped projections 112 are what is called fine microneedles. The length of each needle-shaped projection 112 is from about 1 µm through about 5000 µm, and more preferably from about 5 µm through about 3000 µm. The diameter of the needle-shaped projection 112 around the base thereof is in the range of from 2 µm through 5000 µm, and more preferably in the range of from 5 µm through 3000 µm. Also, it is suitable that the plurality of needle-shaped projections 112 be spaced from each other by from about 0.1 mm through about 10 mm. Note that, although FIG. 4 illustrates an example in which the needle-shaped projections 112 have a contour of a conical shape, the needle-shaped projections 112 may be, for example, a polygonal pyramid shape as illustrated in FIG. 11A, or a cross shape. Alternatively, the needle-shaped projections 112 may have such a shape as to become asymmetric from the front end thereof.

The number of the needle-shaped projections 112 is not limited and is from one through several hundreds, with from several projections through about 100 projections being more preferable. The plurality of needle-shaped projections 112 may be arranged orderly or provided at random positions. Alternatively, the plurality of needle-shaped projections 112 at the bottom face may be those formed at one time with one sheet (like a frog) or an assembly of one or a plurality of needle-shaped projection units each having a plurality of needle-shaped projections formed in a row.

Alternatively, the below-described pyramidal needle-shaped projection 22A (see FIG. 11A) or star-like polygonal pyramid shape (see FIG. 11B) may be combined with a conical needle-shaped projection 22. Furthermore, the needle-shaped projection may have a two-stepped shape including a base under the conical front-end portion of the needle-shaped projection (see FIG. 11C). Alternatively, in a plate 31, needle-shaped projections having a plurality of different shapes may be provided.

The lip portion 113 is formed at an outer edge portion of the front-end face (contact face) CF of the plate body 111. In the present embodiment, the lip portion 113 serves as a liquid stopper. The height of the lip portion 113 from the front-end face CF is equal to or shorter (lower) than the length of the needle-shaped projection 112. For example, the height of the lip portion 113 is such a height that the liquid can spread all over the target object; i.e., from about 10 through about 2000 µm.

In the present embodiment, the needle plate 11 including the needle-shaped projection 112 is suitably made of a biocompatible material including a biodegradable resin such as a polyglycolic acid, a polylactic acid, or a polyglycolic acid-polylactic acid copolymer. Nonetheless, the needle plate 11 may be made of a thermoplastic resin such as polypropylene (PP), polyethylene (PE), acrylonitrile butadiene styrene (ABS), polybutylene terephthalate (PBS), polyacetal (POM), polyethylene terephthalate (PET) polycarbonate (PC), or polyether ether ketone (PEEC).

Meanwhile, the attaching frame 12 includes a plate-supporting portion 121 and an attaching barrel 126.

Specifically, the plate-supporting portion 121 is a barrel with a top face, including a round plate 122 to serve as the top face for housing and an outer barrel 123 to serve as a side wall for housing, and the bottom face thereof forms a recessed space for housing. In the bottom face of the round plate 122 to serve as the top face for housing, the outer edge portion to serve as the boundary with the outer barrel 123 is an annular groove 124.

In assembling the needle-shaped projection-including part 1, the outer periphery of the needle plate 11 is fitted to the inside of the outer barrel 123 of the attaching frame 12. By this fitting, in the present embodiment, the other face of the plate body 111 of the needle plate 11 is closely attached to one face of the round plate 122 of the attaching frame 12. As illustrated in FIG. 5A, in the present embodiment, in a state where the needle plate 11 is attached to the attaching frame 12, the front-end face LT of the lip portion 113 in the needle-shaped projection-including part 1 projects to the outside (lower side) relative to the bottom end ST of the outer barrel 123.

Moreover, a hole is formed at the center of the plate-supporting portion 121. This hole serves as a flow path 125 through which the content passes in the needle-shaped projection-including part 1.

Meanwhile, the attaching barrel 126 has such a cylindrical shape that one end thereof is open and the other end thereof is coupled to the back face of the plate-supporting portion 121. The inside of the barrel serves as the fitting hole 127. The other end of the attaching barrel 126 is a perforated bottom face 128 of the attaching barrel 126.

Also, the sizes of the flow paths (holes) as illustrated in FIG. 4 are in the following order from larger one: the fitting hole 127 > the flow path 125 > the discharge hole 114, as an example. However, the sizes of the holes are not limited to any particular size. With this configuration, a tapered shape of the front barrel portion 84 becomes gradually narrower at the front thereof relative to the attaching barrel 126 when fitting the front barrel portion 84 of the syringe 80 into the attaching barrel 126 in order to attach the needle-shaped projection-including part 1 to the syringe 80. As a result, sealing property is secured by virtue of fitting between the tapers of the side faces. Note that, when the taper of the front barrel portion 84 has narrowed such that further insertion of the front barrel portion 84 is stopped, as illustrated in FIG. 2, a front end 84T of the front barrel portion 84 may contact the perforated bottom face 128 for the fitting hole 127 and the flow path 125.

FIG. 5A and FIG. 5B are views for describing how the needle-shaped projection-including part 1 according to the first embodiment operates for the target object O.

By pushing the syringe barrel 81 of the syringe 80 to the target object O, such as skin, the target object O is punctured by the plurality of needle-shaped projections 112 (see FIG. 5A). At this time, the lip portion 113 of the outer edge portion of the contact face CF of the needle-shaped projection-including part 1 contacts the target object O.

Subsequently, in response to pushing of the pusher 85 of the syringe 80, the liquid L reserved in the syringe 80 flows into the needle-shaped projection-including part 1, and the liquid spreads in a space defined by the target object O, the lip portion 113, and the front-end face CF of the plate body 111 and enters the skin through holes formed by the plurality of needle-shaped projections 112. In other words, the liquid penetrates through the gaps between the surfaces of the needle-shaped projections 112 and the skin (see FIG. 5B).

After injection, for a predetermined time (less than one second through about 30 minutes, preferably about several seconds through about several minutes), the needle-shaped projection-including part 1 is maintained in a state of being in contact with the target object O via the syringe 80.

After the predetermined time has passed, the syringe 80 is taken away from the target object O to withdraw the needle-shaped projections 112 from the target object O.

As described above, in the present invention, the liquid having a beauty effect on skin as the target object O spreads on the skin inside the lip portion 113, and also penetrates the target object O through the holes formed by the needle-shaped projections 112. Therefore, it is possible to penetrate active ingredients of the liquid from both outside and inside the target object O. For example, when the target object O is skin, it is possible to deliver beauty ingredients of the liquid from both outside and inside the skin.

### <Second embodiment of needle-shaped projection-including part>

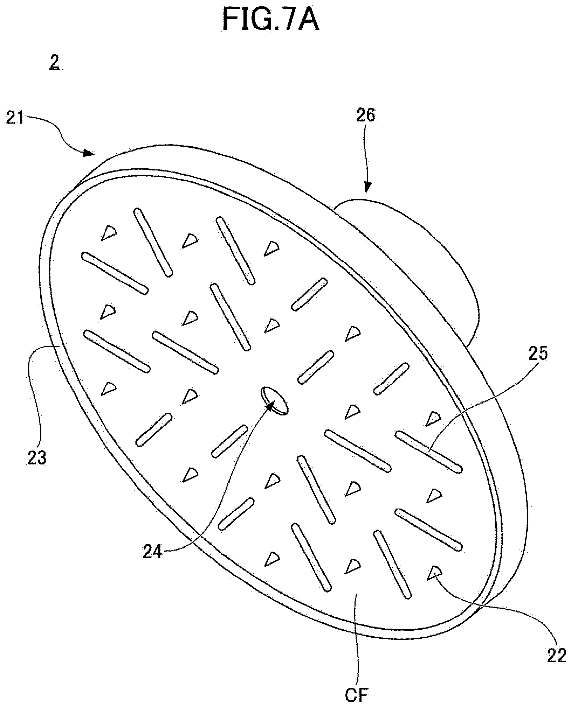

Next, referring to FIG. 6 to FIG. 7B, a needle-shaped projection-including part according to a second embodiment will be described.

FIG. 6 is a view of a needle-shaped projection-including part 2 according to the second embodiment of the present invention. FIG. 7A is a perspective view of the bottom face of the needle-shaped projection-including part 2 of FIG. 6. FIG. 7B is a perspective view of the top face of the needle-shaped projection-including part 2 of FIG. 6.

The above-described needle-shaped projection-including part 1 is an assembled structure (assembly structure) of two members: the attaching frame 12 and the needle plate 11. Nonetheless, the needle-shaped proj ection-including part may be one portion having an attaching function to the front barrel portion of the syringe and a puncturing function.

In the present embodiment, the needle-shaped projection-including part 2 includes a needle plate 21 and an attaching barrel 26 in an integrated manner.

The needle-shaped projection-including part 2 of an integrated type according to the present embodiment is suitably made of a biocompatible material including a biodegradable resin such as a polyglycolic acid, a polylactic acid, or a polyglycolic acid-polylactic acid copolymer. Nonetheless, the needle-shaped proj ection-including part 2 may be made of a thermoplastic resin such as polypropylene (PP), polyethylene (PE), acrylonitrile butadiene styrene (ABS), polybutylene terephthalate (PBS), polyacetal (POM), polyethylene terephthalate (PET) polycarbonate (PC), or polyether ether ketone (PEEC).

The needle plate 21 having a circular flat-plate shape is provided with a plurality of needle-shaped projections 22 and a lip portion 23. The needle plate 21 has a discharge hole 24 at the center thereof.

The height of the lip portion 23 from the front-end face CF is equal to or lower (shorter) than the height of the needle-shaped projection 22.

The attaching barrel 26 has a fitting hole 27. With this configuration, a portion between the inner peripheral face of the fitting hole 27 and the outside of the discharge hole 24 is a perforated bottom face 28 of the attaching barrel 26. In a state where the attaching barrel 26 is engaged with the front barrel portion of the syringe 80, the content L out of the flow path of the front barrel portion 84 hits and passes through the perforated bottom face 28. In order for the liquid to move smoothly, the perforated bottom face 28 may have a tapered or curved shape towards the discharge hole 24 (i.e., a through-hole).

Moreover, in the present embodiment, puncture amount-adjusting ribs 25 are provided to regulate the depth by which the needle-shaped projections 22 enter the target object. Note that, the height of the puncture amount-adjusting ribs 25 is lower than the height of the needle-shaped projections 22, and is lower than the height of the lip portion 23 or nearly equal to the height of the lip portion 23.

In the present embodiment as well, by integrally providing the lip portion 23 and the bottom face of the needle plate 21, the liquid having an effect on the target object spreads on the skin inside the lip portion 23, and also penetrates the target object through the holes formed by the needle-shaped projections 22. Therefore, it is possible to penetrate active ingredients of the liquid from both outside and inside the target object. For example, when the target object is skin, it is possible to penetrate beauty ingredients of the liquid from both outside and inside the skin.

### <Modified example of the plate shape in the needle-shaped projection-including part>

FIG. 8A and FIG. 8B are views of a needle-shaped projection-including part according to a modified example of the second embodiment. FIG. 8A is a perspective view of the bottom face thereof, and FIG. 8B is a perspective view of the top face thereof.

In the present modified example, a needle plate 21A that is to contact the target object is a triangle with round corners. Note that, although FIG. 8A and FIG. 8B illustrate an example in which the contour of the needle plate 21A is a triangle, the contour of the needle plate 21A may be another polygon such as a tetragon or a pentagon.

The needle-shaped projection-including part of the present modified example has the polygonal needle plate 21A. Thus, for example, when a liquid is applied to linear angular portions and curved portions, such as the vicinity of a human nose and the outline of a face, by using the needle-shaped projection-including part of the present modified example, it becomes easier to press the needle-shaped projection-including part against the target object.

The above describes the example in which the needle plate 21 (21A) is a circle or a triangle with round corners. However, the needle plate 21 (21A) may have any shape, such as an elliptical shape, an oval shape, a polygon including a tetragon, a tetragon with round corners, or a comma shape.

Note that, the circular needle plate 21 in the above-described needle-shaped projection-including part 2 is suitable for applications to a portion with a large area or a portion with a small number of concave and convex portions, in the target object. Thus, it is suitable to appropriately select the shape of a plate in accordance with applications or sizes.

### <Modified example of central hole>

The above describes an example in which the number of central holes as the discharge hole is one. However, the number of the holes may or may not be one.

FIG. 9 is a view of a modified example of the discharge hole of the needle-shaped projection-including part of the present invention. In the present modified example, by providing reinforcing plates to divide the discharge hole, four discharge holes 24A are formed. Provision of the reinforcing plates 241 can secure strength even if the size of the discharge hole 24A is made large.

Also, in the discharge hole 24A of FIG. 9, which is provided with the reinforcing plates 241, the flow rate to the target object can be reduced even if the discharge hole 24A has the same diameter as that of the discharge hole 24 of FIG. 7A. The optimum value of the flow rate to the target object per time varies with properties of the contained liquid. Thus, in accordance with a specification, whether to provide the reinforcing plate 241, and how many to divide the hole into by increasing or decreasing the number of the reinforcing plates 241 are appropriately determined.

Note that, FIG. 8A to FIG. 9 illustrate examples in which the needle plate 21 has a polygonal shape and the central hole has a shape according to a modified example, as modified examples of the second embodiment in which the needle plate is integrally formed with the attaching barrel. These modified examples may be applied to the needle-shaped projection-including parts 1 and 1A of a two-body type, according to the first embodiment and the third embodiment. Alternatively, these modified examples may be applied to the below-described needle-shaped projection-including part 3 that is free of an attaching barrel and is made only of a plate to be attached to an injector.

### <Third embodiment of needle-shaped projection-including part>

The above describes an example in which the needle plate has one discharge hole at the center thereof. However, the number of the discharge holes may be one or more, and the position of the discharge hole may or may not be at the center.

FIG. 10 is a cross-sectional perspective view of a needle-shaped projection-including part 1A according to the third embodiment. In the present embodiment, in a needle plate 11A, the positions of vertically penetrating discharge holes 114a and 114b are not at the center, and a plurality of discharge holes are provided. In order for the liquid from the flow path 125 in the attaching barrel 126 to be moved to the discharge holes 114a and 114b apart from the center, a gap for the liquid to pass through is provided between an attaching frame 12A and a plate body 111A of the needle plate 11A.

In order to achieve the gap, in the present embodiment, the front-end face of a round plate 122A of a plate-supporting portion 121A of the attaching frame 12A is provided with an annular projected portion 129 at a certain distance from the center. When the needle plate 11A is attached to the attaching frame 12A, the front end of the annular projected portion 129 contacts the back-end face (rear face) BF of the plate body 111A, whereby a region surrounded by the annular projected portion 129 and the back-end face BF of the plate body 111A becomes a partitioned gap space. When the liquid enters the gap space from the flow path 125 of the attaching frame 12A, a liquid reservoir is formed. The liquid can flow from the liquid reservoir into the discharge holes 114a and 114b.

Therefore, in the present embodiment, the positions of the discharge holes 114a and 114b, provided in the plate body 111A to serve as a plate, are set so as to be closer to the center than is the position of the annular projected portion 129 of the attaching frame 12A.

In the needle-shaped projection-including part 1A in the present embodiment, the liquid from the attaching barrel 126 flows through the flow path 125 at the center of the perforated bottom face 128 and spreads in the gap space on the top face of the plate body 111A as a liquid reservoir, followed by entering the plurality of discharge holes 114a and 114b.

When the liquid that has entered the plurality of discharge holes 114a and 114b is expelled outside the front-end face CF of the plate body 111A, the liquid spreads on the target object towards the inner periphery of the lip portion 113 and also penetrates the target object through holes formed by the needle-shaped projections 112, in the same manner as described above. Therefore, it is possible to penetrate active ingredients of the liquid from both outside and inside the target object.

In the present embodiment, the plurality of discharge holes 114a and 114b, apart from the center, and the gap space form a two-step liquid reservoir on the rear face side and on the front-end face side of the plate body 111A. This makes the liquid easily spread outside. Therefore, even if the liquid is applied to the target object where the liquid does not readily spread from the center to the lip portion 113 as the outer peripheral edge due to roughness of the surface or projections on the surface, it is possible to sufficiently spread the liquid from the center to the outside since there are the discharging holes 114a and 114b at the positions from the center to the lip portion 113.

The number of the discharge holes 114a and 114b is two or more. In the present configuration, increasing the number of discharge holes can more enhance the effect of spreading from the center to the outside.

Moreover, in the present configuration, in order for the liquid to be more efficiently moved from the center to the outside in the gap space between the plate body 111A and the round plate 122A, by using as a flow path a portion having the shortest path through which the liquid flows from the center to the discharge holes 114a and 114b, a band-shaped recessed portion may be formed in the back-end face BF of the plate body 111A.

### <Modified example of needle-shaped projections>

The above-described first to third embodiments describe an example in which the needle-shaped projections 22 (112) have a contour of a conical shape. However, the needle-shaped projections may have a polygonal pyramid shape or another needle shape (e.g., a star shape or a cross shape). Also, needle-shaped projections that are hollow needles can be used instead of the needle-shaped projections that are solid needles.

FIG. 11A to FIG. 11C are views of modified examples of the needle-shaped projections of the needle-shaped projection-including part of the present invention. Specifically, FIG. 11A illustrates a needle-shaped projection 22A having a polygonal pyramid shape. FIG. 11B illustrates a needle-shaped projection 22B having a conical pentagram shape. FIG. 11B illustrates a two-step needle-shaped projection 22C of a base and a front-end portion.

The needle-shaped projection 22A having a polygonal pyramid shape as illustrated in FIG. 11A and the needle-shaped projection 22B having a conical pentagram (five-pointed star) shape as illustrated in FIG. 11B have sharp-edge sides and more readily puncture the target object. For example, these shapes are advantageous in puncture in thickened skin. Note that, although FIG. 11A illustrates a trigonal pyramid as a polygonal pyramid, other shapes such as a tetragonal pyramid and a pentagonal pyramid are possible. Likewise, although FIG. 11B illustrates a conical five-pointed star shape as an example of a conical star-shaped polygon, other shapes such as a six-pointed star and a seven-pointed star are possible.

As illustrated in FIG. 11C, in the two-step needle-shaped projection 22C in which a base portion (base) 222 is provided under a top needle 221 having a conical shape, provision of the base portion 222 can overcome difficulty in puncture due to deformation of skin. Note that, the shape of the top needle 221 or the base portion 222 in the two-step needle-shaped projection 22C as illustrated in FIG. 11C is one example, and other shapes are possible. Note that, the base portion 222 does not puncture the target object but does push and stretch the target object around the top needle 221. Therefore, provision of the base portion 222 can overcome difficulty in puncture due to deformation of skin.

Also, as illustrated in FIG. 11A, in one needle plate 21, the needle-shaped projections 22 having a conical shape and the needle-shaped projections 22A having a polygonal pyramid shape may be included in combination. Moreover, the needle-shaped projections 22B and 22C as illustrated in FIG. 11B and FIG. 11C may be included in combination with the needle-shaped projections 22, 22A, 22B, and 22C having other shapes.

Also, the needle-shaped projections 22 may have different heights. Moreover, the surface of the needle-shaped projection 22 may be provided with slits, fine grooves, concave and convex portions, etc. In this case, it is possible to reduce the quantity of the liquid used or to partially increase the quantity of the liquid used. It is also possible to promote penetration of the liquid through holes in skin formed by puncture.

Note that, the modified examples of the needle-shaped projections as illustrated in FIG. 11A to FIG. 11C may be applied to the needle-shaped projection-including part 1 of a two-body type, according to first to third embodiments. Alternatively, these modified examples may also be applied to the below-described needle-shaped projection-including part 3 that is free of an attaching barrel and is made only of a plate to be attached to an injector. Also, the needle-shaped projections 22A and 22B having the shapes as illustrated in FIG. 11A and FIG. 11B may be applied to the below-described fourth embodiment.

### <Fourth embodiment of needle-shaped projection-including part>

The above describes an example in which the lip portion in the needle plate is provided at the outer peripheral edge of the plate body. However, a lip portion to serve as a liquid stopper may be provided at an outer peripheral edge of the front-end face of the base portion.

Here, referring to FIG. 12 and FIG. 13, a fourth embodiment in which the base portion is provided with the lip portion will be described. FIG. 12 is a cross-sectional perspective view of a needle-shaped projection-including part 5 according to the fourth embodiment of the present invention. FIG. 13 is a view of a state where a liquid is injected into the target object from a needle plate 51 of the needle-shaped projection-including part 5 according to the fourth embodiment.

In the present embodiment, the shape of an attaching frame 12B of the needle-shaped projection-including part 5 is nearly equal to that of the attaching frame 12A of the third embodiment. However, the shape of the needle plate 51 is different from the configurations of the above-described first to third embodiments.

The needle plate 51 of the present embodiment is provided with one or a plurality of base portions 512 that project from one face F1 of a plate body 511. The front-end diameter (d); i.e., the diameter of a front-end face CF2, which is an upper base face of the circular truncated cone shape of the base portion 512, is, for example, from 100 µm through 1500 µm and particularly preferably from 300 µm through 600 µm.

The plurality of base portions 512 are each provided with one or a plurality of fine needle-shaped projections 513 that project from the front-end face CF2 of the base portions 512 and with a lip portion 514 that is formed at the outer edge portion of the front-end face CF2 of the base portions 512.

The needle-shaped projection 513 of the present embodiment are what are called fine microneedles. The length (b) of each of the needle-shaped projections 513 is from about 1 µm through about 5000 µm, and more preferably from about 5 µm through about 3000 µm. The diameter of the needle-shaped projection 513 around the base thereof is in the range of from 2 µm through 5000 µm, and more preferably in the range of from 5 µm through 3000 µm. Also, the pitch (c) between the tops of the plurality of needle-shaped projections 513 is, for example, from 1 mm through 10 mm. It is suitable that the plurality of needle-shaped projections 513 be spaced from each other by more preferably from about 2 mm through about 5 mm.

The height of the lip portion 514 is shorter than or equal to the length (b) of the needle-shaped projection 513, and is, for example, from about 1 µm through about 5000 µm, and more preferably from about 5 µm through about 3000 µm. Also, the thickness of the lip portion 514 is from about 0.05 mm through about 5 mm.

Also, the total height (a+b) of the height (a) of the base portion 512 and the height (b) of the needle-shaped projection 513 is from about 600 µm through 5000 µm. Also, it is suitable that the value of a ratio ((a+b)/c) be 0.1 or more and 2.8 or less, where the ratio ((a+b)/c) is a ratio of the total of the height (a) of the base portion 512 and the height (b) of the needle-shaped projection 513, relative to the pitch (c) of the needle-shaped projections.

The plate body 511 and the plurality of base portions 512 in the needle plate 51 have a discharge hole 515 that vertically penetrates from the other face F2 of the plate body 511 to the contact face CF2 that is the front-end face of the base portions 512.

In the present embodiment, in the needle plate 51, a plurality of vertically penetrating discharge holes 515 are provided at positions other than the center. Therefore, in order for the liquid from the flow path 125 in the attaching barrel 126 to be moved to the plurality of discharge holes 515 apart from the center, a gap for the liquid to pass through is provided between the attaching frame 12B and the other face F2 of the plate body 511 of the needle plate 51.

In order to achieve the gap, in the present embodiment, the front-end face of a round plate 122B of a plate-supporting portion 121B of the attaching frame 12B is provided with an annular projected portion 129 at a certain distance from the center and outside the discharge hole 515 that is the closest to the outer periphery. When the needle plate 51 is attached to the attaching frame 12B, the front end of the annular projected portion 129 contacts the other face F2 that is the rear face of the plate body 511, whereby a region surrounded by the annular projected portion 129 and the other face F2 of the plate body 511 becomes a partitioned gap space. When the liquid enters the gap space from the flow path 125 of the attaching frame 12B, a liquid reservoir is formed. The liquid can flow from the liquid reservoir into the plurality of discharge holes 515.

In the present embodiment, since the discharge holes 515 are provided so as to penetrate the base portions 512, the position of the outermost base portion 512 in the plate body 511 is set so as to be closer to the center than is the position of the annular projected portion 129 of the attaching frame 12B.

In the needle-shaped projection-including part 5 in the present embodiment, the liquid from the attaching barrel 126 flows through the flow path 125 at the center of the perforated bottom face 128 and, as illustrated in FIG. 13, spreads in the gap space on the top face of the plate body 511 as a liquid reservoir, followed by entering the plurality of discharge holes 515.

When the liquid passes through the discharge hole 515 penetrating the plate body 511 and the base portion 512, and then reaches the contact face CF2 of the base portion 512 and is discharged, as illustrated in FIG. 13, the liquid spreads in a space defined by the target object O, the lip portion 514, and the contact face CF2 that is the front end of the base portion 512, and the liquid enters the target object O through holes formed by the needle-shaped projections 513.

In the present embodiment, the liquid spreads on the target object O towards the inner periphery of the lip portion 514 and on the contact face CF2 of the base portion 512, and also penetrates the target object O through holes formed by the needle-shaped projections 513. Therefore, it is possible to penetrate active ingredients of the liquid from both outside and inside the target object O.

In the present embodiment, since a liquid reservoir is formed on the front-end side of the base portions 512, the liquid spreads in a region inside the lip portion 514 of each of the base portions 512. Therefore, for example, in the case in which the needle-shaped projection-including part 5 is large in size and the plate body 511 is large in area, when it is desired to apply the active ingredients at dots and pinpoints, the liquid can be applied in such a manner.

Also, in the present embodiment, as illustrated in FIG. 13, by virtue of the height difference between the base portion 512 and the one face F1 of the plate body 511, the lip portion 514 continuous from the base portion 512 pushes and stretches the target object, such as skin, in a small area for puncture of the needle-shaped proj ection 513. Therefore, the needle-shaped proj ections 513 readily puncture the target object O when the surface of the target object has concave and convex portions or the target object is soft. This makes it possible to more reliably apply the liquid from outside and inside.

Meanwhile, in the case of hollow needles, there is a disadvantage that unless all of the needles puncture the target object, the liquid is discharged from the needles that are not puncturing due to the difference in injection resistance. In the present embodiment, however, regardless of whether the needle-shaped projections 513 puncture the target object, the lip portions 514 contact the target object, and thus the injection pressure (resistance) is uniformly applied to regions inside the inner peripheries of the lip portions 514 that are all of the front-end portions. By this, there is an advantage that liquid injectability can be reliably enhanced.

Moreover, in the present configuration, in order for the liquid to be more efficiently moved from the center to the outside in the gap space between the plate body 511 and the round plate 122B, by using as a flow path a portion having the shortest path through which the liquid flows from the center to the plurality of discharge holes 515, a band-shaped recessed portion may be formed in the other face F2 of the plate body 511.

Also, one base portion 512 may be provided with a plurality of needle-shaped projections or a plurality of discharge holes. In that case, in the base portion 512, the liquid discharged from the plurality of discharge holes can spread on the target object O towards the inner periphery of the lip portion 514 and on the contact face CF2 of the base portion 512, and also can penetrate inside the target object O through holes formed by the plurality of needle-shaped projections 513. «Needle-fixable injector»

Next, referring to FIG. 14 and FIG. 15, description will be given to a needle-fixable injector in which the needle-shaped projection-including part of the present invention is mounted. The needle-shaped projection-including part of the present invention is usable, with the needle-shaped projection-including part being mounted inside a needle-fixable injector.

FIG. 14 is an overall cross-sectional view of a syringe device 200 that is a needle-fixable injector 90 in which a needle-shaped projection-including part 3 according to a fifth embodiment of the present invention is mounted. FIG. 15 is an exploded perspective view of the syringe device 200 of FIG. 14.

In the fifth embodiment, the needle-fixable injector 90, to which the needle-shaped projection-including part 3 is to be attached, includes an injector body 91 and a pusher 95. The injector body (injection guide body) 91 of the present embodiment houses liquid content L, and the pusher 95 is inserted into the injector body 91. Specifically, the injector body 91 includes an outer barrel portion 92 housing the content L, and a head (pressing portion, supporting portion) 93 that widens towards the outer periphery in a brim shape at the front-end side.

Referring to FIG. 14 and FIG. 15, the front-end face of the head 93 includes a thin-plate portion 931 to serve as a top face for housing, and an outer peripheral thick portion 932 to serve as a side wall for housing, thereby forming a recessed portion relative to the front-end side. The inner face of the outer peripheral thick portion 932, corresponding to the height difference between the thin-plate portion 931 and the outer peripheral thick portion 932, forms a recessed portion side wall 933. At the center of the thin-plate portion 931 of the head 93, a communication hole 934 is formed.

Also, in the present embodiment, the needle plate 11 is used as the needle-shaped projection-including part 3.

In the present configuration, in assembling the needle-fixable injector 90, the outer periphery of the needle plate 11 is fitted to the inside of the recessed portion side wall 933 on the bottom-face side of the head 93. As illustrated in FIG. 14, in a state where the needle plate 11 is attached to the inj ector body 91, the front-end face LT of the lip portion 113 in the needle plate 11 projects to the outside (lower side) relative to the bottom end ST of the outer peripheral thick portion 932 of the head 93.

Also, the outer peripheral face of the outer barrel portion 92 may be provided with a side-face hole 921, as illustrated in FIG. 14. For example, when the same person increases the quantity of the content to be applied to the target object per use, the content is injected into the outer barrel portion 92 through the side-face hole 921 using, for example, a dropper. At this time, the quantity of the content is set to be below the side-face hole 921.

The back end of the pusher 95 (plunger) is provided with a pushing part 96 that has a widened brim shape or a wing shape extending in two directions. Also, a gasket 97 adheres to the front-end portion of the pusher 95, where the gasket 97 is a thin-plate packing made of rubber or elastomer for preventing liquid leakage and contamination. In the present configuration, the pusher 95, the pushing part 96, and the gasket 97 are pushing parts. Note that, the gasket 97 may or may not be provided.

Moreover, in the present embodiment, the inner periphery of the outer barrel portion 92 defines a hollow portion 94. Note that, in the present embodiment, although FIG. 14 and FIG. 15 illustrate a state where an absorbent 98 such as a sponge is set in the hollow portion 94 of the outer barrel portion 92, the absorbent 98 may or may not be provided.

In the present embodiment, by pushing the pushing part 96 of the pusher 95 in a state where the needle plate 11 is pushed onto the target object, the pusher 95 descends inside the outer barrel portion 92, and the liquid that has been previously injected to the hollow portion 94 through the side-face hole 921 passes through the absorbent 98. Subsequently, the liquid is discharged through the communication hole 934 and the discharge hole 114, and gradually applied to the target object. Note that, when the absorbent 98 is not provided, the liquid previously injected into the hollow portion 94 is immediately discharged through the communication hole 934 and the discharge hole 114, and applied to the target object.

In the present embodiment as well, by integrally providing the lip portion 113 and the bottom face of the needle plate 11, the liquid having an effect on the target object spreads on skin inside the lip portion 113 without leaking into the device, and also penetrates the target object through the holes formed by the needle-shaped projections 112. Therefore, it is possible to penetrate active ingredients of the liquid from both outside and inside the target object. For example, when the target object is skin, it is possible to penetrate beauty ingredients of the liquid from both outside and inside the skin.

### (Modified example 1)

Note that, in the present embodiment as well, it may be possible to employ, as a modified example of the needle plate 11, the needle-shaped projections having the triangular front-end face as illustrated in FIG. 8A and FIG. 8B, the needle-shaped projections having the polygonal pyramid shape, the two-stepped shape, and the conical star-shaped polygon as illustrated in FIG. 11A to FIG. 11C, and the configuration of the discharge hole provided with a lattice as illustrated in FIG. 9.

### (Modified example 2)

Moreover, in the present embodiment, it may be possible to employ, as a needle plate to be attached to the needle-fixable injector 90, the needle plate 11A including a plurality of discharge holes as illustrated in FIG. 10, where the needle plate 11A is spaced from the thin-plate portion 931 of the head 93. In this configuration, by operating the pushing part, the liquid from the communication hole 934 of the head 93 spreads in the gap space of the back-end face BF of the plate body 111A of the needle plate 11A as a liquid reservoir, followed by entering the plurality of discharge holes 114a and 114b. When the liquid that has passed through the discharge holes 114a and 114b is expelled outside the front-end face CF of the plate body 111A, the liquid spreads on the target object towards the inner periphery of the lip portion 113 and also penetrates the target object through holes formed by the needle-shaped projections 112.

### (Modified example 3)

Also, in the present embodiment, it may be possible to employ, as a needle plate to be attached to the needle-fixable injector 90, the needle plate 51 as illustrated in FIG. 12 and FIG. 13, where the needle plate 51 is spaced from the thin-plate portion 931 of the head 93. In this needle plate 51, each of the plurality of base portions 512 is provided with fine needle-shaped projections 513 and a lip portion 514, and the same number of the discharge holes 515 as the base portions 512 are formed, where the discharge holes 515 penetrate the base portions 512 and the plate body 511. In this configuration, by operating the pushing part, the liquid from the communication hole 934 of the head 93 spreads in the gap space on the top face of the plate body 511 of the needle plate 51 as a liquid reservoir, followed by entering the plurality of discharge holes 515. When the liquid that has passed through the discharge holes 515 is expelled outside the plate body 511 and outside the front-end face CF2 of the base portions 512, the liquid spreads on the target object towards the inner periphery of the lip portion 514 of the base portions 512, and also penetrates the target object through holes formed by the needle-shaped projections 513.

### <Sixth Embodiment>

Note that, as another embodiment of the needle-shaped projection-including part, in the needle-shaped projection-including part to be applied to the needle-fixable injector, the injector body and the needle plate may be integrally formed.

FIG. 16 is a view of a needle-shaped projection-including part 4 of a sixth embodiment, in which needle-shaped projections 42 are provided in a head portion 41 that is integrally formed with a barrel portion 46. In the present embodiment, the needle plate 11 as illustrated in the fifth embodiment is integrally formed with a head (pressing portion, supporting portion) 93 that widens towards the outer periphery in a brim shape at the front-end side, whereby the head portion 41 is formed.

The circular flat-plate front end of the head portion 41 is provided with a plurality of needle-shaped projections 42 and a lip portion 43. At the center of the front-end face CF of the head portion 41, a discharge hole 44 is formed.

In the present configuration as well, the height of the lip portion 43 from the plate front-end face CF of the head portion 41 is lower (shorter) than the height of the needle-shaped projection 42.

The barrel portion 46 has a hollow portion 47. With the present configuration, a portion between the inside of the inner peripheral face on the front-end side of the hollow portion 47 and the outside of the discharge hole 44 is a perforated bottom face 48 of the barrel portion 46, and the pushed-out content L hits and passes through the perforated bottom face 48. In order for the liquid to move smoothly, the perforated bottom face 48 may have a tapered or curved shape towards the discharge hole 44.

Moreover, in the present embodiment as well, although not illustrated, puncture amount-adjusting ribs may be provided that are for regulating the depth by which the needle-shaped projections 42 enter the target object. Note that, the height of the puncture amount-adjusting ribs is lower than the needle-shaped projections 42, and is lower than the height of the lip portion 43 or nearly equal to the height of the lip portion 43.

In the present embodiment, by integrally providing the lip portion 43 and the front-end face (bottom face) of the head portion 41, the liquid having an effect on the target object spreads on the target object inside the lip portion 43, and also penetrates the target object through the holes formed by the needle-shaped projections 42. Therefore, it is possible to penetrate active ingredients of the liquid from both outside and inside the target object.

### (Modified example)

Note that, as a modified example of the head portion 41 of the sixth embodiment, it may be possible to employ the plate shape having the triangular front-end face as illustrated in FIG. 8A and FIG. 8B, the needle-shaped projections having the polygonal pyramid shape, the two-stepped shape, and the conical star-shaped polygon as illustrated in FIG. 11A to FIG. 11C, and the configuration of the discharge hole provided with a lattice as illustrated in FIG. 9.

In any of the configurations of the above-described embodiments and modified examples, since the lip portion is integrally formed with the pressing face, a liquid having some beneficial effect on the target object spreads on the target object towards the inner periphery of the lip portion without leaking into the device, and also penetrates the target object through the holes formed by the needle-shaped projections. In other words, with a device including the needle-shaped projection-including part of the present invention, it is possible to retain a liquid composition/cosmetic liquid containing any active ingredient on and in the target object so as not to leak from the gap.

The syringe or the injector to which any of the needle-shaped projection-including parts according to the above-described embodiments of the present invention is attached has been described, with the beauty substance being taken as an example of the content (liquid substance). However, the content may be another substance selected from the group consisting of a cell suspension, a gel-like material, a therapeutic substance, and a diagnostic substance.

The beauty substance includes a typical beauty substance that can be contained in a cosmetic composition, and a beauty substance used for aesthetic medicine. Examples of the typical beauty substance can include, but are not limited to: ascorbic acid or derivatives thereof, tranexamic acid, arbutin, 4-MSK (potassium 4-methoxysalicylate), etc. for skin lightening applications; and retinol, niacinamide, and hyaluronic acid or derivatives thereof, etc. for anti-wrinkle applications.

In addition to the typical beauty substance that can be contained in a cosmetic composition, examples of the beauty substance to be injected can include, but are not limited to an adipocyte like a filler, hyaluronic acid, a botulinum toxin (Botox, Btx), etc. for wrinkle treatments.

Examples of the therapeutic substance include, but are not limited to, antibiotics, anesthetics, analgesics, vaccines, and antibodies.

Moreover, in order to inject, to a human subject, cells in a suspension or a liquid medium as the content housed in a syringe, the needle-shaped projection-including part of the present invention may be used.

Moreover, the content to be housed in a syringe may be a mixture of a cell suspension and a growth factor. Alternatively, the cell suspension may contain a gel-like structure. Such a gel-like structure preferably represents a mixture of extracellular matrix proteins that mimic extracellular environments of different tissues, and further preferably a gel-like structure similar to hyaluronic acid.

Note that, in the above, the syringe or the injector in which the needle-shaped projection-including part 1 of the present invention is mounted has been described taking the human skin as an example of the target object to which the liquid is to be applied. The target object may be, for example, animal skin, or the surface of a plant culm, stem, or leaf. Alternatively, the target object may be tissue such as skin and organs that are taken out of a human subject or an animal subject.

While preferable embodiments of the present invention have been described above in detail, the present invention is not limited to the specific embodiments, and various modifications and changes are possible within the scope of the gist of the embodiments of the present invention described in the scope of claims.

The present international application claims priority to Japanese Patent Application No. 2020-166213, filed September 30, 2020. The contents of Japanese Patent Application No. 2020-166213 are incorporated herein by reference in their entirety.

**[Description of the Reference Numeral]**

| | | | |
|---|---|---|---|
| 1,2,3,4,5 | Needle-shaped projection- including part | 84 | Front barrel portion |
| | | 85 | Pusher (piston) |
| 11 | Needle plate | 86 | Pushing part (piston) |
| 12 | Attaching frame | 87 | Gasket (piston) |
| 22,42 | Needle-shaped projections | 90 | Needle-fixable injector |
| 23,43 | Lip portion | 92 | Outer barrel portion (injection guide body) |
| 24,44 | Discharge hole | | |
| 26 | Attaching barrel | 93 | Head (injection guide body) |
| 41 | Head portion | 95 | Pusher (pushing portion) |
| 51 | Needle plate | 96 | Pushing part (pushing portion) |
| 511 | Plate body | 97 | Gasket (pushing portion) |
| 512 | Base portion | 111,111A | Plate body (plate) |
| 513 | Needle-shaped projection | 112,112A | Needle-shaped projection |
| 514 | Lip portion | 113 | Lip portion |
| 515 | Discharge hole | 114,114a,114b | Discharge hole |
| 61 | Contact barrel portion | 121 | Plate-supporting portion |
| 65 | Ascending/descending barrel portion | 125 | Flow path |
| 80 | Syringe | 126 | Attaching barrel |
| | | 200 | Injector |
| | | L | Content (liquid) |

## Claims

1. A needle-shaped projection-including part, which is configured to inject a liquid to a target object, the needle-shaped projection-including part comprising:
a plate in which a vertically penetrating discharge hole is formed;
one or a plurality of fine needle-shaped projections that project from one face of the plate; and
a lip portion that is formed at an outer edge portion of the one face of the plate,
wherein in response to the liquid being discharged through the discharge hole, the liquid spreads in a space defined by the target object, the lip portion, and the one face of the plate, and the liquid enters the target object through holes formed by the one or plurality of needle-shaped projections.

2. The needle-shaped projection-including part according to claim 1, wherein a height of the lip portion is shorter than or equal to a length of the one or plurality of needle-shaped projections.

3. The needle-shaped projection-including part according to claim 1 or 2, wherein the one face of the plate is provided with a puncture amount-adjusting rib configured to prevent the one or plurality of needle-shaped projections from overly puncturing.

4. The needle-shaped projection-including part according to claim 3, wherein a height of the puncture amount-adjusting rib is constant and is lower than or equal to the height of the lip portion.

5. The needle-shaped projection-including part according to any one of claims 1 to 4, wherein each needle-shaped projection of the one or plurality of needle-shaped projections is a conical shape or a polygonal pyramid shape.

6. A needle-shaped projection-including part, which is configured to inject a liquid to a target object, the needle-shaped projection-including part comprising:
a plate;
one or a plurality of base portions that project from one face of the plate;
one or a plurality of fine needle-shaped projections that project from a front-end face of each of the one or plurality of base portions; and
a lip portion that is formed at an outer edge portion of the front-end face of the base portion,
wherein the plate and the one or plurality of base portions have a discharge hole that vertically penetrates from another face of the plate to the front-end face of the base portion, and
in response to the liquid being discharged through the discharge hole, the liquid spreads in a space defined by the target object, the lip portion, and the front-end face of the base portion, and the liquid enters the target object through holes formed by the one or plurality of needle-shaped projections.

7. The needle-shaped projection-including part according to any one of claims 1 to 6, wherein:
the needle-shaped projection-including part is usable, with the needle-shaped projection-including part being mounted in a syringe barrel;
the needle-shaped proj ection-including part includes an attaching frame into which the plate is to be fitted;
the attaching frame is provided with an attaching barrel that is open so that a front barrel portion of a front end of the syringe barrel is fitted; and
in response to pushing of a piston inserted into the syringe barrel, the liquid is expelled from the attaching barrel and discharged through the discharge hole.

8. The needle-shaped projection-including part according to claim 7, wherein:
one vertically penetrating discharge hole is formed in the plate so as to be on a substantially same straight line with a hollow portion of the attaching barrel in a state where the plate is fitted into and engaged with the attaching frame;
the plate is fitted into the attaching frame so that another face of the plate is closely attached to one face of the attaching frame; and
in response to pushing of the piston inserted into the syringe barrel, the liquid passes through the hollow portion of the attaching barrel and the discharge hole continuous therefrom, and is discharged through the plate.

9. The needle-shaped projection-including part according to claim 7, wherein:
a plurality of vertically penetrating discharge holes are formed in the plate at positions apart from center;
the plate is fitted into the attaching frame so that another face of the plate faces one face of the attaching frame with a predetermined gap; and
in response to pushing of the piston inserted into the syringe barrel, the liquid is expelled from the attaching barrel to the one face of the attaching frame, the liquid moves so as to spread between the another face of the plate and the one face of the attaching frame, and then the liquid is discharged through the discharge holes.

10. The needle-shaped projection-including part according to any one of claims 1 to 5, wherein:
the needle-shaped projection-including part is usable, with the needle-shaped projection-including part being mounted in a syringe barrel;
the needle-shaped projection-including part includes
an attaching barrel integrally formed with another face of the plate, where the attaching barrel is open so that a front barrel portion of a front end of the syringe barrel is fitted and the attaching barrel has a hollow portion in communication with the discharge hole; and
in response to pushing of a piston inserted into the syringe barrel, the liquid is expelled from the attaching barrel and discharged through the discharge hole.

11. The needle-shaped projection-including part according to any one of claims 1 to 6, wherein:
the needle-shaped projection-including part is usable, with the needle-shaped projection-including part being mounted in a needle-fixable injector;
the needle-fixable injector includes
an injection guide body that includes a cylindrical side wall having an injection hole, and a head having a communication hole, where the head is provided at a front end of the side wall, and
a pushing portion that is partially inserted inside the side wall of the injection guide body, where the pushing portion is configured to ascend and descend in the side wall;
the plate of the needle-shaped projection-including part is fitted into and engaged with a front end of the head; and
in response to pushing of the pushing portion and descending of the pushing portion in the side wall after injecting of the liquid into the injection guide body through the injection hole, the liquid passes through the communication hole of the head and the discharge hole of the plate, and is discharged.

12. The needle-shaped projection-including part according to any one of claims 1 to 5, wherein:
the needle-shaped projection-including part is usable, with the needle-shaped projection-including part being integrally formed with a needle-fixable injector;
the needle-fixable injector includes
an injection guide body that includes a cylindrical side wall having an injection hole, and a head portion provided at a front end of the side wall, and
a pushing portion that is partially inserted inside the side wall of the injection guide body, where the pushing portion is configured to ascend and descend in the side wall;
the plate of the needle-shaped projection-including part is integrally formed as a front-end face of the head portion; and
in response to pushing of the pushing portion and descending of the pushing portion in the side wall after injecting of the liquid into the injection guide body through the injection hole, the liquid passes through the discharge hole of the plate in the head portion, and is discharged.
